# EUROPEAN PATENT SPECIFICATION

(11) **EP 3 876 876 B1**
(45) Date of publication and mention of the grant of the patent: **25.01.2023**
(21) Application number: 20723673.8
(22) Date of filing: 10.04.2020
(51) Int. Cl.: A61F 5/00

(54) **DELIVERY SYSTEMS FOR DEVICES WITH UNSUPPORTED STRUCTURE**
LIEFERSYSTEME FÜR VORRICHTUNGEN MIT TRÄGERLOSER STRUKTUR
SYSTÈMES D'ADMINISTRATION POUR DISPOSITIFS À STRUCTURE NON SUPPORTÉE

(30) Priority: 11.04.2019 US 201962832762 P
(43) Date of publication of application: 15.09.2021
(73) Proprietor: Boston Scientific Scimed, Inc., Maple Grove, MN 55311 (US)
(72) Inventor: GILMARTIN, Gary, Foxford, F26 CK30 (IE); TONER, Geraldine A., Lifford Co.Donegal Raphoe (IE); FOLAN, Martyn G., Galway Galway (IE); TUCK, Daniel, Galway (IE); KEATING, Thomas M., Tuam, Co Galway (IE)
(74) Representative: Vossius & Partner Patentanwälte Rechtsanwälte mbB
(86) International application number: PCT/US2020/027712
(87) International publication number: WO 2020/210659

(56) References cited:
- WO-A1-2015/095416
- WO-A1-2017/062753
- US-A1- 2005 192 614

## Description

### FIELD

The present disclosure relates generally to the field of medical devices and medical device delivery. In particular, the present disclosure relates to a medical device, method and delivery system for positioning a medical device with an unsupported structure within a distant and/or tortuous portion of body lumen.

### BACKGROUND

Conventional endoscopic delivery systems used to deliver medical devices (*e*.*g*., stents, clips, etc.) into body lumens and/or passages tend to be rigid to allow for tracking through tortuous anatomies or require a previously positioned ancillary device (*e*.*g*., guidewire, endoscope, etc.) to reach a target location. Certain body lumens may be too inaccessible and/or tortuous to safely and efficiently access the target location using these conventional systems or techniques. This problem tends to be amplified when a substantial portion of the medical device includes an unsupported structure that may lack the requisite rigidity to be delivered without the benefit of a previously positioned ancillary device.
WO 2017/062753 A1 discloses an electroporation device comprising: a shaft defining a first lumen therethrough; a proximal balloon circumferentially attached about a distal portion of the shaft; a middle portion extending distally of the proximal balloon, the middle portion defining a middle portion lumen in communication with the first lumen, the middle portion including one or more electrodes configured to administer electroporation energy, the middle portion including one or more apertures through a wall of the middle portion and in communication with the middle portion lumen, the middle portion having a longitudinally contracted configuration and a longitudinally extended configuration that is longer than the longitudinally contracted configuration; and a distal balloon extending distally of the middle portion.

A variety of advantageous medical outcomes may therefore be realized by the medical devices and medical device delivery systems of the present disclosure.

### SUMMARY

The invention is defined by the features of the independent claims.

In one aspect, the present disclosure relates to a medical device delivery system comprising an elongate member and a medical device disposed within a distal portion of the elongate member. The medical device may include a proximal supported structure and a distal unsupported structure. The medical device may be configured to move from a first position, in which the supported structure is disposed within the distal portion of the elongate member in a first configuration, and a second position in which the supported structure extends beyond a distal end of the elongate member in a second configuration. A plurality of balloons may be disposed within the distal portion of the elongate member and the unsupported structure. A fluid delivery lumen may extend through the elongate member. A proximal end of the fluid delivery lumen may be configured to fluidly connect to a fluid source and a distal end of the fluid delivery lumen may be in fluid communication with the plurality of balloons. Each balloon of the plurality of balloons may be in a concertinaed configuration. The unsupported structure may be in a concertinaed configuration over the plurality of balloons. A distal end of the unsupported structure may be attached to a distal balloon of the plurality of balloons.

In the described and other embodiments, the plurality of balloons may include a proximal balloon, a distal balloon and one or more intermediate balloons extending between the proximal and distal balloons. A proximal end of the proximal balloon may include an opening configured to fluidly receive the distal end of the fluid delivery lumen. A distal end of the distal balloon may be sealed. Adjacent balloons of the plurality of balloons may be attached by a flexible connector. The flexible connector may include a lumen extending therethrough such that adjacent balloons are in fluid communication with each other. The flexible connectors between adjacent balloons may provide fluid communication therebetween along a length of the plurality of balloons. The flexible connector may be configured to provide a flexible junction between adjacent balloons as the plurality of balloons move from the concertinaed configuration to a linearly expanded configuration. A plug may be removably disposed within the lumen of the flexible connector. The plug may be configured to prevent or limit a flow of inflation fluid into one or more of the plurality of balloons until a proximally adjacent balloon is in a linearly expanded configuration. The plurality of balloons may be configured to linearly expand sequentially from a proximal to distal direction. The unsupported structure may move from the concertinaed configuration to a linearly extended configuration as the balloons move from the concertinaed configuration to the linearly expanded configuration. The unsupported structure may linearly extend and be pulled distally along with the sequentially linearly expanding plurality of balloons. The supported structure may be configured to anchor the medical device within a body lumen. The sequentially linearly expanded plurality of balloons may be configured to pull the linearly extended unsupported structure into a distal portion of the body lumen. An inner surface of the unsupported structure may be attached to an outer surface of one or more of the plurality of balloons. The supported structure in the second position may include a flared flange, a cylindrical portion distal to the flared flange and an elastomeric membrane extending between the flared flange and cylindrical portion. The flared flange and cylindrical portion may be configured to anchor the medical device across a pylorus of a patient. The unsupported structure may include an impermeable liner.

In another aspect, the present disclosure relates to a medical device comprising a proximal supported structure and a distal unsupported structure. The unsupported structure may include distal end deployable from an elongate delivery member. A plurality of balloons may be deployable from within the unsupported structure and removably attached to the distal end thereof. The balloons may be configured in a concertinaed configuration, when undeployed, and are inflatable in series distally from the elongate delivery member, when deployed. The unsupported structure may be configured in a concertinaed configuration, when undeployed, and may unfold distally as the balloons are inflated, when deployed.

In the described and other embodiments, the plurality of balloons may include a proximal balloon, a distal balloon and one or more intermediate balloons extending between the proximal and distal balloons. A proximal end of the proximal balloon may include an opening configured to fluidly receive the distal end of the fluid delivery lumen. A distal end of the distal balloon may be sealed. Adjacent balloons of the plurality of balloons may be attached by a flexible connector. The flexible connector may include a lumen extending therethrough such that adjacent balloons are in fluid communication with each other. The flexible connectors between adjacent balloons may provide fluid communication therebetween along a length of the plurality of balloons. The flexible connector may be configured to provide a flexible junction between adjacent balloons as the plurality of balloons move from the concertinaed configuration to a linearly expanded configuration. A plug may be removably disposed within the lumen of the flexible connector. The plug may be configured to prevent or limit a flow of inflation fluid into one or more of the plurality of balloons until a proximally adjacent balloon is in a linearly expanded configuration. The plurality of balloons may be configured to linearly expand sequentially from a proximal to distal direction. The unsupported structure may move from the concertinaed configuration to a linearly extended configuration as the balloons move from the concertinaed configuration to the linearly expanded configuration. The unsupported structure may linearly extend and be pulled distally along with the sequentially linearly expanding plurality of balloons. The supported structure may be configured to anchor the medical device within a body lumen. The sequentially linearly expanded plurality of balloons may be configured to pull the linearly extended unsupported structure into a distal portion of the body lumen. An inner surface of the unsupported structure may be attached to an outer surface of one or more of the plurality of balloons. The supported structure in the second position may include a flared flange, a cylindrical portion distal to the flared flange and an elastomeric membrane extending between the flared flange and cylindrical portion. The flared flange and cylindrical portion may be configured to anchor the medical device across a pylorus of a patient. The unsupported structure may include an impermeable liner.

In yet another aspect, the present disclosure relates to a method of delivering a medical device with a body lumen. The method may include advancing a medical device delivery system into the body lumen. The medical device delivery system may include an elongate member and a medical device. The medical device may be disposed within the elongate member and include a distal unsupported structure. The method may further include introducing an inflation fluid into a proximal balloon of a plurality of balloons such that the proximal balloon moves from a concertinaed configuration to a linearly expanded configuration and a portion of the unsupported structure attached to the plurality of balloons moves from a concertinaed configuration to a linearly extended configuration to distally advance the unsupported structure through the body lumen.

In the described and other embodiments, introducing the inflation fluid into the plurality of balloons may sequentially move the plurality of balloons from the concertinaed configuration to the linearly expanded configuration and the unsupported structure attached to the plurality of balloons may moves from the concertinaed configuration to the linearly extended configuration to sequentially distally advance the unsupported structure. Adjacent balloons of the plurality of balloons may be attached by a flexible connector. The method may further include detaching the linearly expanded configuration of the plurality of balloons from the linearly extended configuration of the unsupported structure.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. In the figures:
FIGS. 1A-1B provide perspective views of a medical device delivery system, according to one embodiment of the present disclosure.
FIGS. 2A-2I illustrate representative steps of a medical device deployed within a body lumen using the medical device delivery system of FIGS. 1A-1B, according to one embodiment of the present disclosure.
FIGS. 3A-3F provide perspective views of a medical device delivery system, according to one embodiment of the present disclosure.

### DETAILED DESCRIPTION

The present disclosure is not limited to the particular embodiments described herein. The terminology used herein is for the purpose of describing particular embodiments only, and is not intended to be limiting beyond the scope of the appended claims. Unless otherwise defined, all technical terms used herein have the same meaning as commonly understood by one of ordinary skill in the art to which the disclosure belongs.

Although embodiments of the present disclosure are described with specific reference to a medical device delivery system for delivering a medical device (*e.g.*, an anti-obesity device) with a supported portion and an unsupported portion across the pylorus and the duodenum and into a portion of the jejunum of a patient, it should be appreciated that such medical device delivery systems may be used to deliver a variety of medical devices (*e.g.*, guidewires, access sheaths, cannulas, stents, catheters, etc.) into a variety of different body lumens and or passageways, including, for example, anal access to the transverse colon, ascending colon or ileum, Roux-en Y procedures, jejuncolic bypass procedures, jejunoileal bypass procedures, gastrectomy procedures, biliopancreatic diversion with duodenal switch (BPD-DS) procedures, gastrojejunostomy procedures, segmental colonic resection procedures, and the like.

As used herein, the singular forms "a," "an," and "the" are intended to include the plural forms as well, unless the context clearly indicates otherwise. It will be further understood that the terms "comprises" and/or "comprising," or "includes" and/or "including" when used herein, specify the presence of stated features, regions, steps elements and/or components, but do not preclude the presence or addition of one or more other features, regions, integers, steps, operations, elements, components and/or groups thereof.

As used herein, the term "distal" refers to the end farthest away from the medical professional when introducing a device into a patient, while the term "proximal" refers to the end closest to the medical professional when introducing a device into a patient.

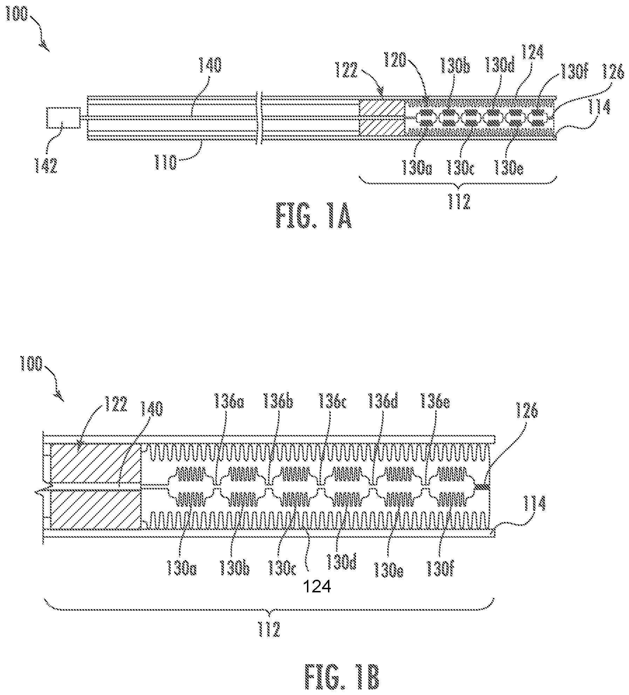

Referring to FIGS. 1A-1B, in one embodiment, a medical device delivery system 100 of the present disclosure may include an elongate member 110 (*e.g.*, delivery catheter, endoscope, etc.) and a medical device 120 disposed (*e.g.*, loaded) within a distal portion 112 of the elongate member 110. In various embodiments, the medical device 120 may include a first (*e.g.*, proximal) supported structure 122 and a second (*e.g.*, distal) unsupported structure 124. The elongate member 110 may be configured to move (*e.g.*, slide, etc.) from a first position (*e.g.*, distally extended) in which the supported structure 122 is disposed within the distal portion 112 of the elongate member 110 in a first configuration (*e.g.*, constrained, non-expanded, non-deployed or delivery configuration, etc.) to a second position (*e*.*g*., proximally retracted) in which the supported structure 122 extends beyond a distal end 114 of the elongate member 110 in a second configuration (*e.g.*, unconstrained, expanded, deployed, etc.).

A plurality of balloons 130 (*e.g.*, non-compliant dilation balloons) may be disposed (*e.g.*, loaded) in a tandem or linear configuration within (*e.g.*, inside) the unsupported structure 124 of the medical device 120 and the distal portion 112 of the elongate member 110. In various embodiments, the plurality of balloons 130 may include a proximal balloon, a distal balloon and one or more intermediate balloons. For example, an embodiment of the present disclosure which includes six balloons in a tandem or linear configuration may include a proximal balloon 130a (*e.g.*, first balloon progressing distally), a distal balloon 130f (*e.g.*, sixth balloon progressing distally) and second 130b, third 130c, fourth 130d and fifth 130e intermediate balloons therebetween. Similarly, and by way of non-limiting example, an embodiment of the present disclosure which includes ten balloons may include a proximal balloon (*e.g.*, first balloon) a distal balloon (*e.g.*, tenth balloon) and second, third, fourth, fifth, sixth, seventh, eighth and ninth balloons therebetween. Each balloon 130a-130f of the plurality of balloons 130 may be disposed within the unsupported structure 124 and within the distal portion 112 of the elongate member 110 in a concertinaed configuration (*e.g.*, collapsed in an accordion-like configuration). In addition, the unsupported structure 124 may be disposed in a concertinaed configuration over the respective outer surfaces of each of the plurality of concertinaed balloons 130a-130f. A distal end of the unsupported structure 124 may be attached to a distal end of the distal balloon 130f (*e.g.*, the distal-most balloon of the plurality of balloons) by a release mechanism 126 (*e.g.*, clip, hook, adhesive, perforated or frangible portion, etc.) as are commonly known in the art. In various additional embodiments, an inner surface of the unsupported structure 124 may be intermittently attached or glued to an outer surface of one or more of the balloons 130a-130f, *e.g.*, using a silicone-based adhesive. Glue may be able to dissolve in the body to break the attachment. Other structure and/or function for detaching the unsupported structure from the balloons are contemplated as desired for a particular application. For example, different possibilities for detachment, include mechanical, chemical and/or electric based attachments, wherein the attachment between the unsupported structure and balloons may be broken by applying force to overcome a mechanical attachment, or an electrical current or chemical substance may act to sever the attachment.

A fluid delivery lumen 140 (*e.g.*, inflation lumen) may extend through a full length of the elongate member 110, with a proximal end of the fluid delivery lumen configured to fluidly connect to a fluid source 142 (*e.g.*, syringe, pump, etc.) and a distal end of the fluid delivery lumen in fluid communication with an opening in the proximal end of the proximal balloon 130a (*e.g.*, the proximal-most balloon of the plurality of balloons) to deliver an inflation fluid (*e.g.* air, water, saline, etc.) from the fluid source 142 sequentially (*e.g.*, from the proximal to distal direction) into each of the plurality of balloons 130a-130f.

In one embodiment, the proximal end of the proximal balloon 130a may include an opening in fluid communication with the distal end of a fluid delivery lumen 140 (as discussed above) and the distal balloon 130f may include a closed/sealed distal end. In various embodiments, adjacent balloons 130a-130f of the plurality of balloons 130 may be attached to each other by a flexible connector 136a, 136b, 136c, 136d, 136e (*e.g.*, section of flexible tubing). Each of the flexible connectors 136a-136e may include an open lumen extending therethrough such that the adjacent balloons, and therefore each of the plurality of balloons including the proximal and distal balloons, are in fluid communication with each other. In addition to providing fluid communication between each of the plurality of balloons, the flexible connectors 136a-136e may also provide a flexible junction or pivot point between adjacent balloons to carry the unsupported structure 124 through challenging navigation paths, such as a tortuous anatomy, as the balloons 130a-130f sequentially move (*e.g.*, in a proximal to distal direction) from a concertinaed configuration to a linearly expanded (*e.g.*, elongated) configuration. For example, as each balloon 130a-130f sequentially moves from the concertinaed configuration to the linearly expanded configuration, the corresponding portion of the unsupported structure 124 may also move from the concertinaed configuration to a linearly extended configuration to "track" (*e.g.*, pulled or carried distally) through the body lumen along with the sequentially linearly expanded configuration of the balloons. In embodiments of the present disclosure in which the concertinaed unsupported structure 124 and respective underlying concertinaed balloon(s) 130a-130f are attached (*e.g.*, glued using a silicone-based adhesive), the linearly and/or outward expansion of the balloons may provide sufficient force (*e.g.*, radial and/or longitudinal force) to break or otherwise disrupt the attachment point(s) to release the unsupported structure from the balloon(s).

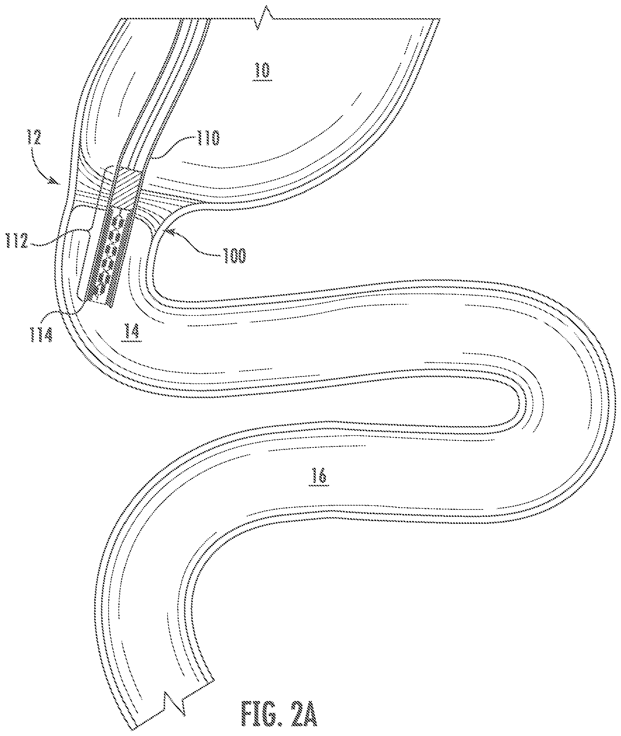

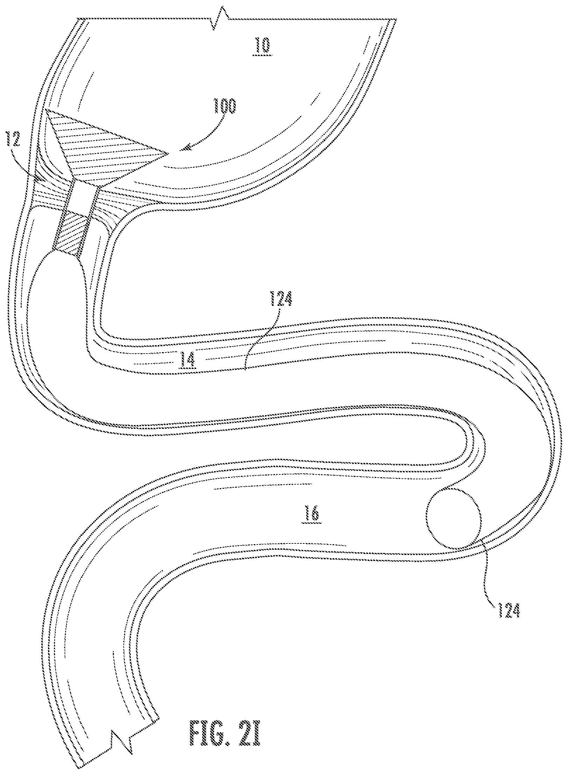

Referring to FIGS. 2A-2I, in use and by way of example, the elongate member 110 of a medical device delivery system 100 of the present disclosure may be advanced through the esophagus and into a the stomach 10 of a patient, and the distal portion 112 of the elongate member 110 positioned across the pylorus 12 with the distal end 114 of the elongate member 110 extending into the duodenum 14 (FIG. 2A).

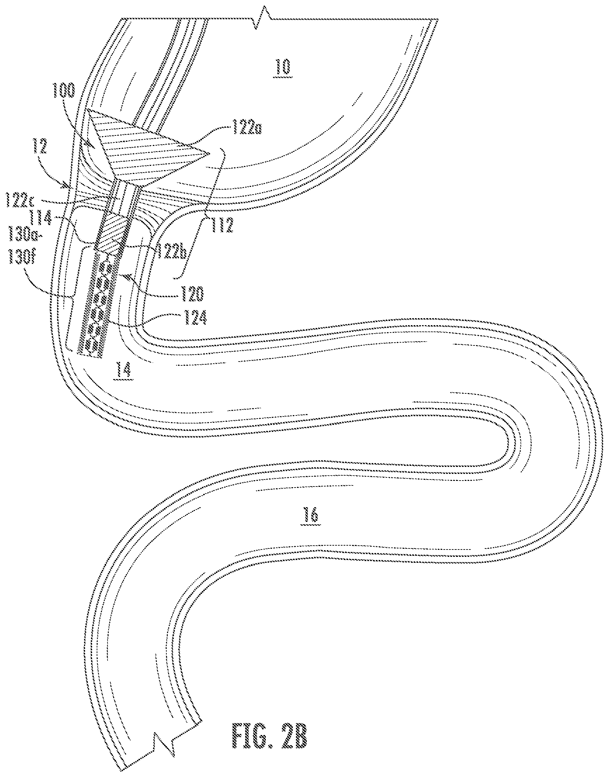

Referring to FIG. 2B, the distal portion 112 of the elongate member 110 may then be proximally retracted to advance (*e.g.*, extend) the medical device 120 beyond the distal end 114 of the elongate member 110 (*e.g.*, medical device 120 is deployed within the patient) with the supported structure positioned/anchored across the pylorus 12 in the second configuration and the unsupported structure 124 and balloons 130a-130f disposed within the duodenum 14 in the concertinaed configurations. In one embodiment, in the second configuration the supported structure 122 may include a self-expanding flared flange 122a (*e.g.*, disposed within the stomach 10), a self-expanding cylindrical portion 122b distal to the flared flange 122a (*e.g.*, disposed within the duodenum 14) and an elastomeric membrane 122c extending between, and connecting, the flared flange 122a and the cylindrical portion 122b (*e.g.*, disposed across the pylorus 12). In various embodiments, the elastomeric membrane 122c may extend around an entire inner and/or outer surface of the flared flange 122a and/or cylindrical portion 122b. The unsupported structure 124 may include a sleeve or liner formed from or otherwise including an impermeable membrane (*e*.*g*., elastomeric membrane, etc.) attached to or integrally formed with an inner and/or outer surface of the cylindrical portion 122b. In various embodiments, the elastomeric membrane 122c and the unsupported structure 124 may include the same or different materials. In various additional embodiments, the elastomeric membrane 122c and the unsupported structure 124 may be formed from or otherwise include a single continuous (*e*.*g*., unitary) sheet of the elastomeric membrane.

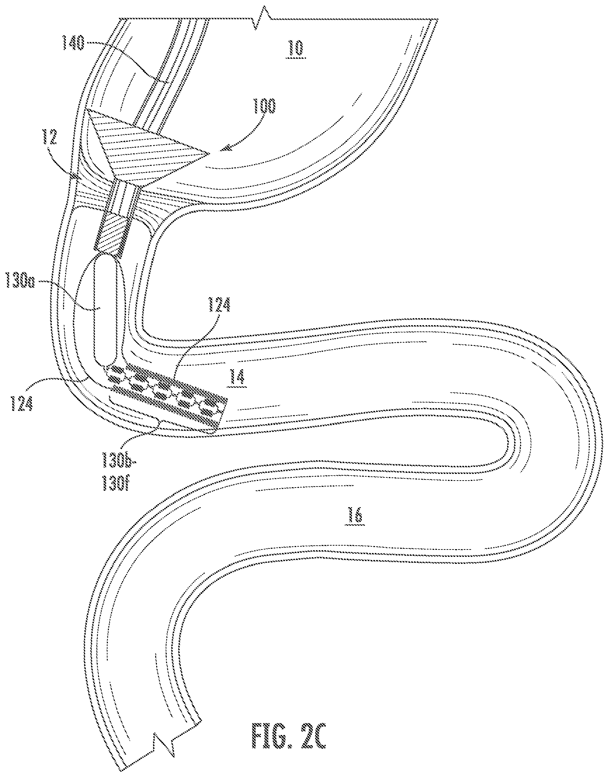

Referring to FIG. 2C, with the supported structure 122 of the medical device anchored across the pylorus 12, inflation fluid may be introduced from the fluid source 142 (FIG. 1A) through the fluid delivery lumen 140 (FIG. 1A) and into the proximal balloon 130a to move the proximal balloon 130a from the concertinaed configuration to a linearly expanded configuration. The concertinaed portion of the unsupported structure 124 disposed over and/or in contact with the proximal balloon 130a may also move from the concertinaed configuration to a linearly extended configuration as the linearly expanded proximal balloon 130a pulls the unsupported structure 124 distally through the duodenum 14 and towards the jejunum 16.

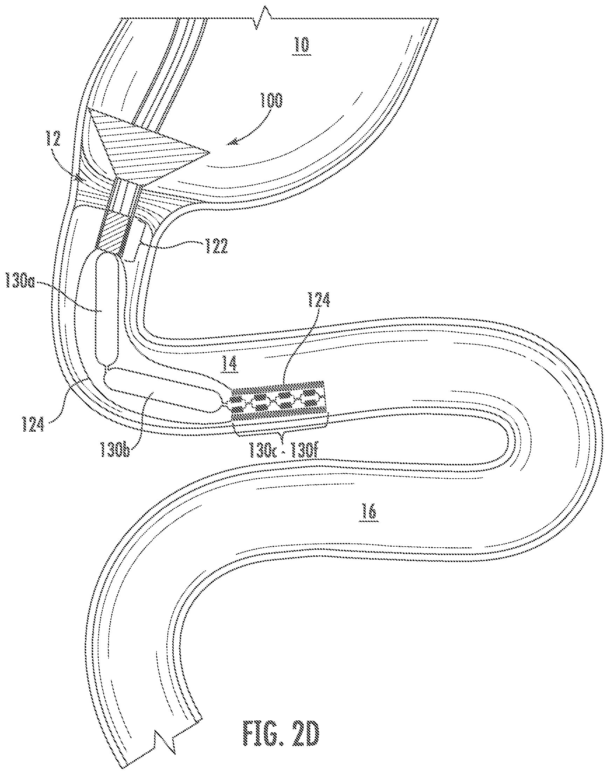

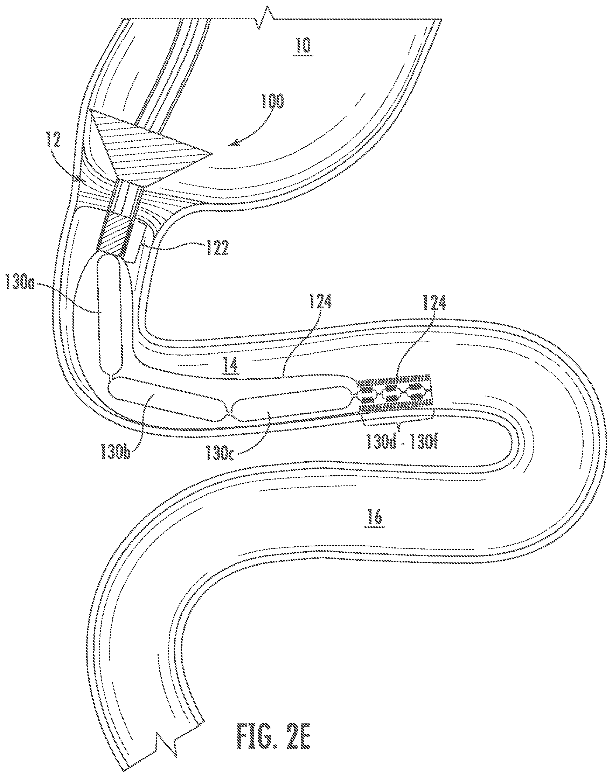

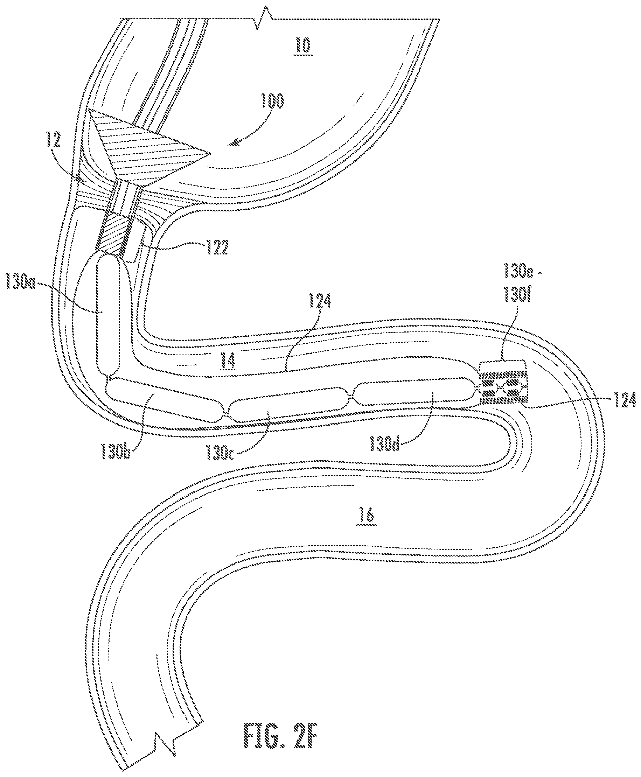

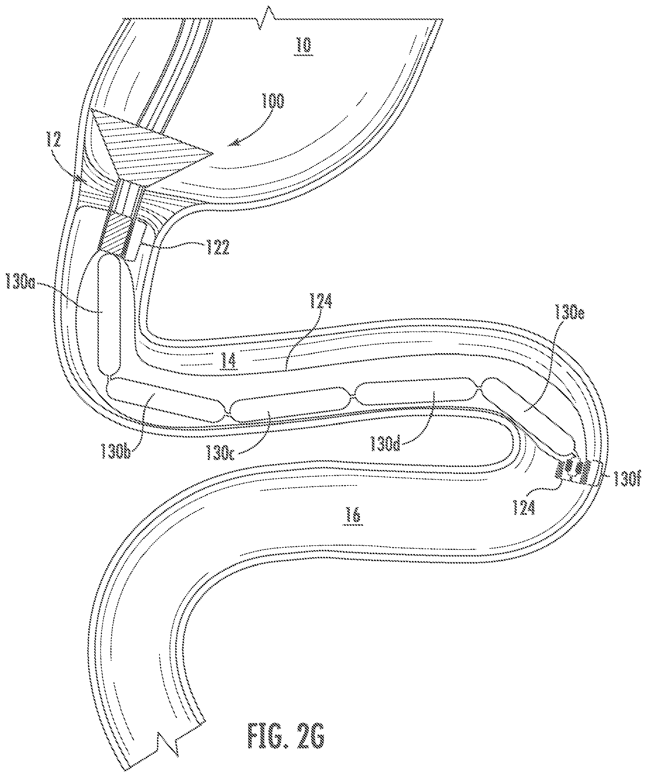

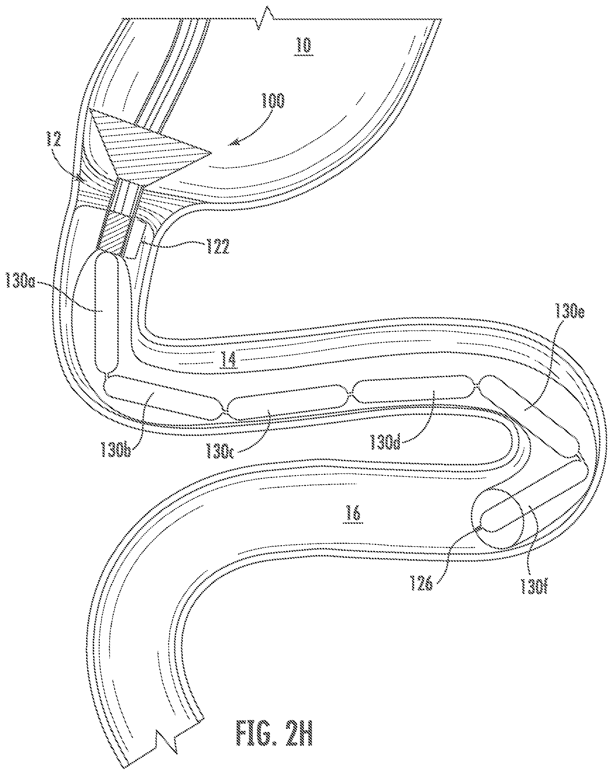

Referring to FIGS. 2D-2H, inflation fluid may continue to be introduced through the fluid delivery lumen 140 to sequentially inflate balloons 130b (FIG. 2D), 130c (FIG. 2E), 130d (FIG. 2F) and 130e (FIG. 2H) and 130f (FIG. 2H), and sequentially/incrementally move the corresponding concertinaed portions of the unsupported structure 124 disposed over and/or in contact each of the respective balloons 130b-130f 124 to a linearly extended configuration to track (*e.g.*, pull, carry, etc.) the unsupported structure 124 further into the duodenum 14 towards the jejunum 16. Referring to FIGS. 2G-2H as exemplary embodiments, the flexible connector 136d connecting balloons 130d and 130e and the flexible connector 136e connecting balloons 130e and 130f may allow those balloons and the respective portion of the unsupported structure 124 to atraumatically bend or curve through a curved or tortuous portion of the duodenum 14.

Referring to FIG. 2H, with the distal balloon 130f in the linearly expanded configuration, an attachment point between the distal end of the distal balloon 130f and the distal end of the unsupported structure 124 may be separated by activating a release mechanism 126, such as described above. Referring to FIG. 21, the balloons 130a-130f may then be removed from within the patient, either in the linearly expanded configuration or in a deflated configuration, and the balloons 130a-130f and elongate member 110 removed from the patient. Alternatively, the balloons 130a-130f may remain in place within the unsupported structure, either in linearly expanded configuration or in a deflated configuration.

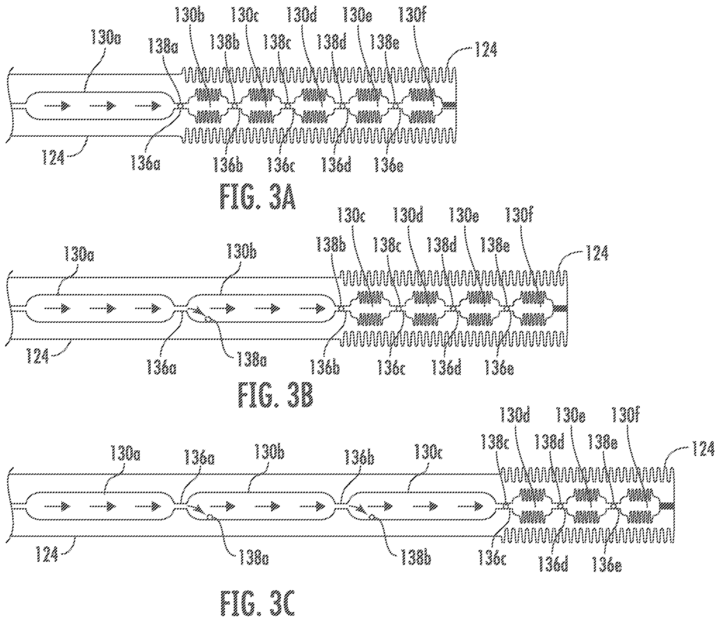

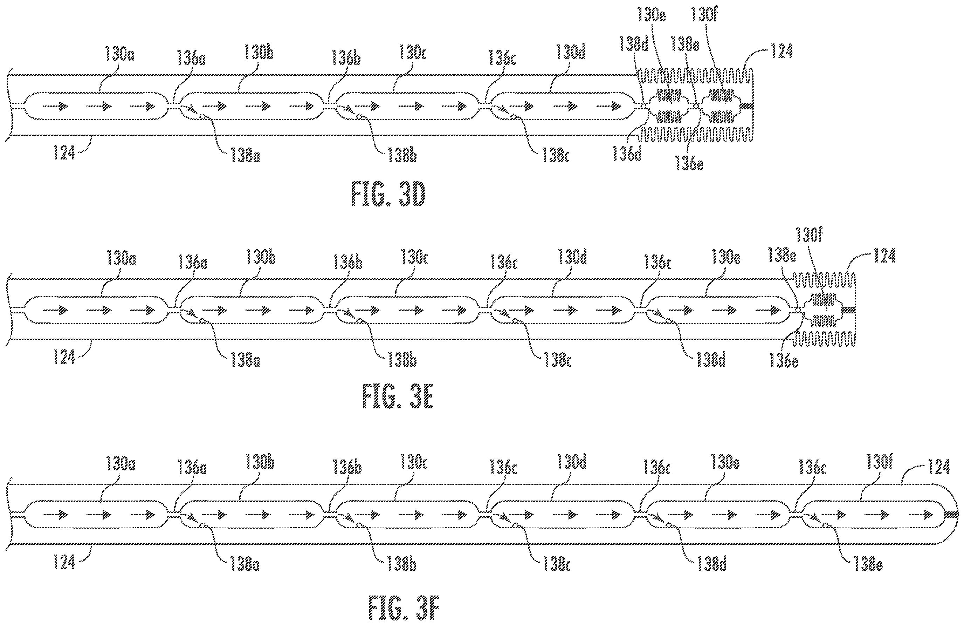

Referring to FIGS. 3A-3F, in one embodiment, a plug 138a, 138b, 138c, 138d, 138e (*e.g.*, stoppers, corks, stopping balls, etc.) may be disposed within the lumens of the respective flexible connectors 136a, 136b, 136c, 136d, 136e. The plugs 138a-138e may be configured to prevent or limit the flow of inflation fluid into respective balloons 130b-130f until the immediately adjacent proximal balloon is fully inflated (*e.g.*, linearly expanded), thereby further ensuring that the balloons sequentially expand in a proximal to distal direction to carry/pull the unsupported structure distally within the duodenum 14 towards the jejunum 16. For example, with the proximal balloons 130a in the linearly expanded (*e.g.*, fully inflated) configuration (FIG. 3A) the internal pressure within the proximal balloon 130a may reach a threshold level to eject the plug 138a from within the lumen of the flexible connector 136a to allow inflation fluid to flow into and linearly expand the immediately adjacent balloon 130b (FIG. 3B). Inflation fluid may continue to be introduced to through the linearly expanded balloons to sequentially linearly expand the immediately adjacent balloons 130c (FIG. 3C), 130d (FIG. 3D), 130e (FIG. 3E) and 130f (FIG. 3F). In various additional embodiments, the flexible connectors 136a-136e may be twisted to close their respective internal lumens to prevent or limit the flow of inflation fluid into respective balloons 130b-130f until the immediately adjacent balloon is fully inflated.

In various embodiments, each of the balloons 130a-130f may include a length of approximately 40.0 mm and a width of approximately 6.0 mm in the linearly expanded configuration. In addition, the flexible connectors may include a length of approximately 5.0 mm and a lumen with an inner diameter of approximately 2.0 mm. Although the medical device delivery system 100 of the present disclosure includes six balloons with substantially identical sizes/shapes, in various embodiments the number of balloons and/or their respective lengths and diameters when linearly expanded may vary, including repeating or non-repeating patterns of long and short balloons, *e*.*g*., depending on the specific medical device and body lumen. For example, the unsupported structure 124 of the medical device 120 of the present disclosure may include an impermeable membrane extending approximately 600 mm past the pylorus and into a distal portion of the jejunum to prevent or limit absorption of nutrients across the wall of the duodenum and jejunum.

Although the medical device delivery system 100 of the present disclosure includes an inflation lumen configured to deliver inflation fluid (*e.g.*, gas, liquid, gel, etc.) into the proximal-most balloon for sequential inflation of the balloons in a proximal to distal direction, in various embodiments the inflation lumen may extend over/along an outer surface of the plurality of balloons to deliver inflation fluid into the distal-most balloon for sequential inflation of the balloons in a distal to proximal direction.

In various additional embodiments, a medical device delivery system of the present disclosure may be configured to deliver a guidewire (rather than an unsupported structure of a medical device) within a body lumen. For example, the medical device delivery system may further include a contiguous lumen extending through the full length of the elongate member and through each of the plurality of balloons. The balloons may then be sequentially linearly expanded as discussed above to provide a contiguous guidewire lumen through which a guidewire may then be introduced. The balloons may then be retracted over the guidewire and removed from the patient, and a subsequent medical device tracked along/over the guidewire. Alternatively, the guidewire may be disposed within the contiguous lumen as the balloons are linearly expanded.

In various additional embodiments, the balloons 130a-130f of the present disclosure may be formed from or otherwise include a variety of flexible and non-compliant materials (*e*.*g*., silicone, polyethylene terapthelate (PET), nylon, hard rubbers, etc.) such that the balloons do not over-expand within the body lumen. In addition, or alternatively, rather than including a plurality of balloons, a medical device delivery system of the present disclosure may include a single elongate balloon disposed in a concertinaed configuration within the unsupported structure. The single elongate balloon may incrementally move from the concertinaed configuration to a plurality of incrementally increasing partially expanded configurations to track the unsupported structure through a body lumen.

In various additional embodiments, the balloons and/or unsupported structure may be collapsed, compacted and/or folded in a non-concertinaed configuration when in an undeployed (*e*.*g*., non-expanded or non-extended) state. In various embodiments, one or more of the balloons may be formed from a variety of non-compliant materials such that each balloon may move to a single pre-determined expanded configuration. Alternatively, one or more of the balloons may be formed from a variety of compliant materials such that each balloon may move to a variety of different expanded configurations. Although the balloons of the present disclosure are depicted including an expanded configuration with a cross-sectional dimension less than a corresponding inner dimension of the unsupported structure, in various embodiments one or more of the balloons may include an expanded configuration substantially equal to an inner dimension of the unsupported structure. In various embodiments, the balloons and/or the unsupported structure may be formed from a sufficiently pliable or flexible material(s) to allow the balloons and unsupported structure to bend or flex when advanced through tortuous body passages.

In various additional embodiments, fewer than all of the plurality of balloons may be linearly expanded when delivering the unsupported structure into a body lumen. For example, one or more of the balloons may remain in the concertinaed configuration once the unsupported structure is determined to have reached a desired portion of the body lumen. In various embodiments, one or more of non-expanded balloons may be moved to the expanded configuration during a subsequent medical procedure.

In various additional embodiments, the present disclosure is not limited to anchoring a medical device across the pylorus and delivering the unsupported structure into the duodenum. By way of non-limiting example, a medical device of the present disclosure may be anchored across an esophagus and the unsupported structure delivered through the stomach and across the pylorus into the duodenum.

All of the devices and/or methods disclosed and claimed herein can be made and executed without undue experimentation in light of the present disclosure. While the devices and methods of this disclosure have been described in terms of preferred embodiments, it may be apparent to those of skill in the art that variations can be applied to the devices and/or methods and in the steps or in the sequence of steps of the method described herein without departing from the concept and scope of the disclosure. All such similar substitutes and modifications apparent to those skilled in the art are deemed to be within the scope and concept of the disclosure as defined by the appended claims.

## Claims

1. A medical device delivery system (100), comprising:
an elongate member (110);
a medical device (120) disposed within a distal portion (112) of the elongate member (110), the medical device (120) comprising a proximal supported structure (122) and a distal unsupported structure (124);
the medical device (120) configured to move from a first position, in which the supported structure (122) is disposed within the distal portion (112) of the elongate member (110) in a first configuration, to a second position in which the supported structure (122) extends beyond a distal end (114) of the elongate member (110) in a second configuration;
a plurality of balloons (130) configured to be disposed within the distal portion (112) of the elongate member (110) and the unsupported structure (124); and
a fluid delivery lumen (140) extending through the elongate member (110), a proximal end of the fluid delivery lumen (140) configured to fluidly connect to a fluid source (142) and a distal end of the fluid delivery lumen (140) in fluid communication with the plurality of balloons (130);
wherein each balloon (130) of the plurality of balloons (130) is configured to be in a concertinaed configuration;
wherein the unsupported structure (124) is configured to be in a concertinaed configuration over the plurality of balloons (130); and
wherein a distal end of the unsupported structure (124) is attached to a distal balloon (130f) of the plurality of balloons (130).

2. The medical device delivery system (100) of claim 1, wherein the plurality of balloons (130) includes a proximal balloon (130a), the distal balloon (130f) and one or more intermediate balloons (130b, 130c, 130d, 130e) extending between the proximal and distal balloons (130a, 130f).

3. The medical device delivery system (100) of any of claims 1-2, wherein a proximal end of the proximal balloon (130a) includes an opening configured to fluidly receive the distal end of the fluid delivery lumen (140).

4. The medical device delivery system (100) of any of claims 1-3, wherein adjacent balloons (130) of the plurality of balloons (130) are attached by a flexible connector ( 136a-e).

5. The medical device delivery system (100) of claim 4, wherein the flexible connector includes a lumen extending therethrough such that adjacent balloons (130) are in fluid communication with each other.

6. The medical device delivery (100) system of any of claims 4-5, wherein the flexible connectors (136a-e) between adjacent balloons provide fluid communication therebetween along a length of the plurality of balloons (130).

7. The medical device delivery system (100) of any of claims 4-6, wherein the flexible connectors (136a-e) are configured to provide a flexible junction between adjacent balloons (130) as the plurality of balloons (130) move from the concertinaed configuration to a linearly expanded configuration.

8. The medical device delivery system (100) of any of claims 4-7, further comprising a plug (138a-138e) removably disposed within the lumen of the flexible connector, wherein the plug (138a-138e) is configured to prevent or limit a flow of inflation fluid into one or more of the plurality of balloons (130) until a proximally adjacent balloon (130) is in a linearly expanded configuration.

9. The medical device delivery system (100) of any of claims 1-8, wherein the plurality of balloons (130) is configured to linearly expand sequentially from a proximal to distal direction.

10. The medical device delivery system (100) of claim 9, wherein the unsupported structure (124) moves from the concertinaed configuration to a linearly extended configuration as the balloons (130) move from the concertinaed configuration to the linearly expanded configuration.

11. The medical device delivery system (100) of claim 10, wherein the unsupported structure (124) is linearly extendable, and is extendable distally in response to linear expansion of the plurality of balloons (130).

12. The medical device delivery system (100) of any of claims 9-11, wherein the supported structure (122) is configured to anchor the medical device (120) within a body lumen.

13. The medical device delivery system (100) of any of claims 1-12, wherein the supported structure (122) in the second position includes a flared flange (122a), a cylindrical portion (122b) distal to the flared flange (122a) and an elastomeric membrane (122c) extending between the flared flange (122a) and cylindrical portion (122b).

14. The medical device delivery system (100) of claim 13, wherein the flared flange (122a) and cylindrical portion (122b) are configured to anchor the medical device (120) across a pylorus (12) of a patient.

15. The medical device delivery system (100) of any of claims 1-14, wherein the unsupported structure (124) includes an impermeable liner.

## Patentansprüche

1. Medizinvorrichtungszuführungssystem (100), aufweisend:
ein längliches Element (110);
eine medizinische Vorrichtung (120), die in einem distalen Abschnitt (112) des länglichen Elements (110) angeordnet ist, wobei die medizinische Vorrichtung (120) eine proximale gestützte Struktur (122) und eine distale ungestützte Struktur (124) aufweist;
wobei die medizinische Vorrichtung (120) konfiguriert ist, sich zu bewegen von einer ersten Position, in der die gestützte Struktur (122) in einer ersten Konfiguration in dem distalen Abschnitt (112) des länglichen Elements (110) angeordnet ist, zu einer zweiten Position, in der sich die gestützte Struktur (122) in einer zweiten Konfiguration über ein distales Ende (114) des länglichen Elements (110) hinaus erstreckt;
mehrere Ballons (130), die konfiguriert sind, innerhalb des distalen Abschnitts (112) des länglichen Elements (110) und der ungestützten Struktur (124) angeordnet zu sein; und
ein Fluidzuführungslumen (140), das sich durch das längliche Element (110) erstreckt, wobei ein proximales Ende des Fluidzuführungslumens (140) konfiguriert ist, an eine Fluidquelle (142) fluidisch angeschlossen zu werden, und ein distales Ende des Fluidzuführungslumens (140) in Fluidkommunikation mit den mehreren Ballons (130) ist;
wobei jeder Ballon (130) der mehreren Ballons (130) konfiguriert ist, in einer zusammengefalteten Konfiguration zu sein; und
wobei die ungestützte Struktur (124) konfiguriert ist, in einer zusammengefalteten Konfiguration über den mehreren Ballons (130) zu sein; und
wobei ein distales Ende der ungestützten Struktur (124) an einem distalen Ballon (130f) der mehreren Ballons (130) angebracht ist.

2. Medizinvorrichtungszuführungssystem (100) nach Anspruch 1, wobei die mehreren Ballons (130) einen proximalen Ballon (130a), den distalen Ballon (130f) und einen oder mehrere dazwischenliegende Ballons (130b, 130c, 130d, 130e), die sich zwischen dem proximalen und dem distalen Ballon (130a, 130f) erstrecken, aufweisen.

3. Medizinvorrichtungszuführungssystem (100) nach einem der Ansprüche 1 bis 2, wobei ein proximales Ende des proximalen Ballons (130a) eine Öffnung aufweist, die konfiguriert ist, das distale Ende des Fluidzuführungslumens (140) fluidisch zu empfangen.

4. Medizinvorrichtungszuführungssystem (100) nach einem der Ansprüche 1 bis 3, wobei benachbarte Ballons (130) der mehreren Ballons (130) mittels eines flexiblen Verbinders (136a-e) befestigt sind.

5. Medizinvorrichtungszuführungssystem (100) nach Anspruch 4, wobei der flexible Verbinder ein hindurchgehendes Lumen aufweist, so dass benachbarte Ballons (130) miteinander in Fluidkommunikation sind.

6. Medizinvorrichtungszuführungssystem (100) nach einem der Ansprüche 4 bis 5, wobei die flexiblen Verbinder (136a-e) zwischen benachbarten Ballons eine Fluidkommunikation entlang einer Länge der mehreren Ballons (130) bereitstellen.

7. Medizinvorrichtungszuführungssystem (100) nach einem der Ansprüche 4 bis 6, wobei die flexiblen Verbinder (136a-e) konfiguriert sind, eine biegsame Verbindungsstelle zwischen benachbarten Ballons (130) bereitzustellen, wenn sich die mehreren Ballons (130) aus der zusammengefalteten Konfiguration in eine linear ausgedehnte Konfiguration bewegen.

8. Medizinvorrichtungszuführungssystem (100) nach einem der Ansprüche 4 bis 7, desweiteren aufweisend einen Pfropf (138a-138e), der lösbar in dem Lumen des flexiblen Verbinders angeordnet ist, wobei der Pfropf (138a-138e) konfiguriert ist, ein Hineinfließen von Blähfluid in einen oder mehrere Ballons (130) solange zu verhindern oder zu begrenzen, bis ein proximal benachbarter Ballon (130) in einer linear ausgedehnten Konfiguration ist.

9. Medizinvorrichtungszuführungssystem (100) nach einem der Ansprüche 1 bis 8, wobei die mehreren Ballons (130) konfiguriert sind, sich nacheinander von einer proximalen zu einer distalen Richtung linear auszudehnen.

10. Medizinvorrichtungszuführungssystem (100) nach Anspruch 9, wobei sich die ungestützte Struktur (124) aus der zusammengefalteten Konfiguration in eine linear ausgestreckte Konfiguration bewegt, wenn die Ballons (130) sich aus der zusammengefalteten Konfiguration in die linear ausgedehnte Konfiguration bewegen.

11. Medizinvorrichtungszuführungssystem (100) nach Anspruch 10, wobei in Antwort auf die lineare Ausdehnung der mehreren Ballons (130) die ungestützte Struktur (124) linear ausstreckbar ist und distal ausstreckbar ist.

12. Medizinvorrichtungszuführungssystem (100) nach einem der Ansprüche 9 bis 11, wobei die gestützte Struktur (122) konfiguriert ist, die medizinische Vorrichtung (120) in einem Körperhohlraum zu verankern.

13. Medizinvorrichtungszuführungssystem (100) nach einem der Ansprüche 1 bis 12, wobei die gestützte Struktur (122) in der zweiten Position einen aufgeweiteten Flansch (122a), einen zylindrischen Abschnitt (122b) distal zu dem aufgeweiteten Flansch (122a) und eine Elastomer-Membran (122c), die sich zwischen dem aufgeweiteten Flansch (122a) und dem zylindrischen Abschnitt (122b) erstreckt, aufweist.

14. Medizinvorrichtungszuführungssystem (100) nach Anspruch 13, wobei der aufgeweitete Flansch (122a) und der zylindrische Abschnitt (122b) konfiguriert sind, die medizinische Vorrichtung (120) über einen Pylorus (12) eines Patienten zu verankern.

15. Medizinvorrichtungszuführungssystem (100) nach einem der Ansprüche 1 bis 14, wobei die ungestützte Struktur (124) ein impermeables Innenfutter aufweist.

## Revendications

1. Système de fourniture de dispositif médical (100), comprenant:
un élément allongé (110);
un dispositif médical (120) disposé à l'intérieur d'une partie distale (112) de l'élément allongé (110), le dispositif médical (120) comprenant une structure supportée proximale (122) et une structure non supportée distale (124);
le dispositif médical (120) configuré pour se déplacer d'une première position, dans laquelle la structure supportée (122) est disposée à l'intérieur de la partie distale (112) de l'élément allongé (110) dans une première configuration, à une seconde position dans laquelle la structure supportée (122) s'étend au-delà d'une extrémité distale (114) de l'élément allongé (110) dans une seconde configuration;
une pluralité de ballonnets (130) configurés pour être disposés à l'intérieur de la partie distale (112) de l'élément allongé (110) et de la structure non supportée (124); et
une lumière de fourniture de fluide (140) s'étendant à travers l'élément allongé (110), une extrémité proximale de la lumière de fourniture de fluide (140) configurée pour se connecter de manière fluidique à une source de fluide (142) et une extrémité distale de la lumière de fourniture de fluide (140) en communication fluidique avec la pluralité de ballonnets (130);
dans lequel chaque ballonnet (130) de la pluralité de ballonnets (130) est configuré pour être dans une configuration en accordéon;
dans lequel la structure non supportée (124) est configurée pour être dans une configuration en accordéon au-dessus de la pluralité de ballonnets (130); et
dans lequel une extrémité distale de la structure non supportée (124) est liée à un ballonnet distal (130f) de la pluralité de ballonnets (130).

2. Système de fourniture de dispositif médical (100) selon la revendication 1, dans lequel la pluralité de ballonnets (130) inclut un ballonnet proximal (130a), le ballonnet distal (130f) et un ou plusieurs ballonnets intermédiaires (130b, 130c, 130d, 130e) s'étendant entre les ballonnets proximal et distal (130a, 130f).

3. Système de fourniture de dispositif médical (100) selon l'une quelconque des revendications 1 à 2, dans lequel une extrémité proximale du ballonnet proximal (130a) inclut une ouverture configurée pour recevoir de manière fluidique l'extrémité distale de la lumière de fourniture de fluide (140).

4. Système de fourniture de dispositif médical (100) selon l'une quelconque des revendications 1 à 3, dans lequel des ballonnets (130) adjacents de la pluralité de ballonnets (130) sont liés par un connecteur flexible (136a-e).

5. Système de fourniture de dispositif médical (100) selon la revendication 4, dans lequel le connecteur flexible inclut une lumière s'étendant à travers celui-ci de telle sorte que des ballonnets (130) adjacents sont en communication fluidique entre eux.

6. Système de fourniture de dispositif médical (100) selon l'une quelconque des revendications 4 à 5, dans lequel les connecteurs flexibles (136a-e) entre les ballonnets adjacents assurent une communication fluidique entre eux le long d'une longueur de la pluralité de ballonnets (130).

7. Système de fourniture de dispositif médical (100) selon l'une quelconque des revendications 4 à 6, dans lequel les connecteurs flexibles (136a-e) sont configurés pour assurer une jonction flexible entre des ballonnets (130) adjacents lorsque la pluralité de ballonnets (130) passe de la configuration en accordéon à une configuration expansée linéairement.

8. Système de fourniture de dispositif médical (100) selon l'une quelconque des revendications 4 à 7, comprenant en outre un bouchon (138a-138e) disposé de manière amovible à l'intérieur de la lumière du connecteur flexible, dans lequel le bouchon (138a-138e) est configuré pour empêcher ou limiter un écoulement de fluide de gonflage dans un ou plusieurs de la pluralité de ballonnets (130) jusqu'à ce qu'un ballonnet (130) adjacent de manière proximale soit dans une configuration expansée linéairement.

9. Système de fourniture de dispositif médical (100) selon l'une quelconque des revendications 1 à 8, dans lequel la pluralité de ballonnets (130) est configurée pour s'expanser linéairement successivement d'une direction proximale à distale.

10. Système de fourniture de dispositif médical (100) selon la revendication 9, dans lequel la structure non supportée (124) passe de la configuration en accordéon à une configuration en extension linéairement lorsque les ballonnets (130) passent de la configuration en accordéon à la configuration expansée linéairement.

11. Système de fourniture de dispositif médical (100) selon la revendication 10, dans lequel la structure non supportée (124) est extensible linéairement, et est extensible de manière distale en réponse à l'expansion linéaire de la pluralité de ballonnets (130).

12. Système de fourniture de dispositif médical (100) selon l'une quelconque des revendications 9 à 11, dans lequel la structure supportée (122) est configurée pour ancrer le dispositif médical (120) à l'intérieur d'une lumière corporelle.

13. Système de fourniture de dispositif médical (100) selon l'une quelconque des revendications 1 à 12, dans lequel la structure supportée (122) dans la seconde position inclut un rebord évasé (122a), une partie cylindrique (122b) distale par rapport au rebord évasé (122a) et une membrane élastomère (122c) s'étendant entre le rebord évasé (122a) et la partie cylindrique (122b).

14. Système de fourniture de dispositif médical (100) selon la revendication 13, dans lequel le rebord évasé (122a) et la partie cylindrique (122b) sont configurés pour ancrer le dispositif médical (120) sur un pylore (12) d'un patient.

15. Système de fourniture de dispositif médical (100) selon l'une quelconque des revendications 1 à 14, dans lequel la structure non supportée (124) inclut une doublure imperméable.
